# EUROPÄISCHE PATENTANMELDUNG

(11) **EP 0 900 824 A1**
(43) Veröffentlichungstag der Anmeldung: **10.03.1999**
(21) Anmeldenummer: 98116197.9
(22) Anmeldetag: 27.08.1998
(51) Int. Cl.: C08K 5/3437

(54) **4-Hydroxychinolin-3-carbonsäure-Derivate als Lichtschutzmittel**

(30) Priorität: 04.09.1997 DE 19738616
(71) Anmelder: Clariant GmbH, 65929 Frankfurt am Main (DE)
(72) Erfinder: Stährfeldt, Thomas, 86356 Neusäss (DE); Mehrer, Mathias, Dr., 86456 Gablingen (DE)

(57) **Zusammenfassung**

Die vorliegende Erfindung bezieht sich auf die Verwendung von Verbindungen der Formel (I) 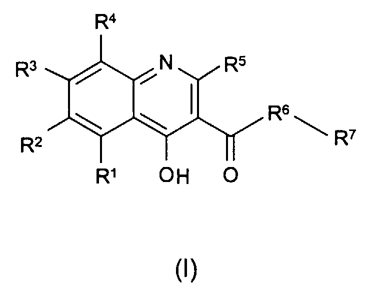 wobei die Substituenten die in der Beschreibung definierte Bedeutung aufweisen, als Stabilisator für organisches Material gegen den schädlichen Einfluß von Licht, Sauerstoff und Wärme.

## Beschreibung

Es ist bekannt, daß organische Materialien durch Licht, Strahlung, Wärme oder Sauerstoff geschädigt werden. Es gibt bereits viele Druckschriften, die Verbindungen zur Stabilisierung von organischem Material gegen die genannten Einflüsse beschreiben. Es handelt sich dabei meist um Radikalfänger, Hydroperoxidzersetzer, Quencher (Löscher für angeregte Zustände) oder UV-Absorber (vgl. R. Gächter, H. Müller, Plastics Additives Handbook, 3rd Ed., Hanser Verlag, München 1990, S. 133 ff). Im Zusammenhang mit UV-Absorbem handelt es sich in der Regel um Verbindungen auf der Basis von 2-Hydroxybenzophenon, 2-Hydroxyphenylbenzotriazol, Zimtsäureester und Oxanilide (vgl. R. Gächter, H. Müller, Plastics Additives Handbook, 3rd Ed., Hanser Verlag, München 1990, S. 181 ff). Auch die Klasse der o-Hydroxy-substituierten Triphenylpyrimidine kann als separater UV-Stabilisatortyp angesehen werden (DE-A-4416809).

Die genannten Verbindungsklassen weisen oft spezifische Nachteile auf, die neben der erwünschten stabilisierenden Wirkung auftreten. Besonders bezüglich Farbverhalten, Wechselwirkung mit Pigmenten, Verträglichkeit der verschiedenen Stabilisatoren untereinander und mit dem zu stabilisierenden Material, Resistenz gegen Chemikalien und Wasser (Hydrolyseempfindlichkeit), Lagerstabilität, Migrationsverhalten und Verbesserung der Stabilisierung gegen die schädigenden Einflüsse von Wärme und Licht, im besonderen im Langzeitgebrauch, besteht ein großer Bedarf nach neuen Stabilisatorklassen.

Derivate der 4-Hydroxychinolin-3-carbonsäure der Formel (I) sind seit einiger Zeit im Zusammenhang mit pharmazeutischen Anwendungen bekannt (vgl. z. B. E. Schroetter et al, Pharmazie (1977), 32(4), 223-5).

Die Anwendungen beziehen sich auf die Wirkung von Verbindungen der Formel (I) gegen Bakterien, Parasiten, Tumore, eitrige Geschwüre, gastrointestinale Fehlfunktionen und als Enzyminhibitoren. Eine stabilisierende Wirkung von Verbindungen der Formel (I) auf organisches Material, insbesonders ihr Potential als UV-Absorber, ist nicht bekannt.

Verfahren zur Herstellung von Verbindungen der Formel (I) sind bekannt z. B. aus Organic Syntheses Coll. Vol. III, S. 274; Houben-Weyl, Heterarene II, Teil 1, Band E7a, S. 343 ff.

Es wurde nun überraschend gefunden, daß Verbindungen der Formel (I) organisches Material hervorragend gegen die schädlichen Einflüsse von Licht, Wärme und Sauerstoff stabilisieren. Die Substanzen (I) besitzen Absorptionsbanden im UV-Bereich mit Extinktionskoeffizienten bis zu Werten von über 20000.

Es handelt sich hierbei um eine neue Stabilisatorklasse, die organisches und anorganisches Material effektiv gegen die schädigenden Einflüsse von Wärme und Licht schützt. Die Verbindungen dieser neuen Stabilisatorklasse können auf die äußere Schicht des zu stabilisierenden Materials aufgebracht oder dem Material beigemischt werden.

Gegenstand der Erfindung ist somit die Verwendung von Verbindungen der Formel (I) als Stabilisator für organisches Material gegen den schädlichen Einfluß von Licht, Sauerstoff und Wärme, 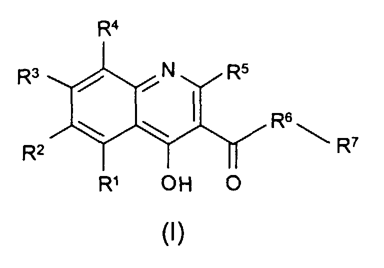 wobei
R¹ bis R⁵ unabhängig voneinander Halogen, H, NO₂, CF₃, CN; C₁-C₂₀-, vorzugsweise C₁-C₁₀-, insbesondere C₁-C₄-Alkyl, -S-Alkyl oder -O-Alkyl; C₆-C₁₄-, vorzugsweise C₆-C₁₀-Aryl, -S-Aryl oder -O-Aryl; C₅-C₁₃-, vorzugsweise C₅-C₁₀-Heteroaryl; C₇-C₂₆-, vorzugsweise C₇-C₁₃-Alkylaryl, -S-Alkylaryl oder -O-Alkylaryl bedeuten.
   Zwei der Reste R¹ bis R⁴ können zusammen mit dem Grundkörper einen 5-12-gliedrigen, vorzugsweise einen 5-6-gliedrigen, mit Halogen, NO₂, CN, CF₃, C₁-C₂₀-, vorzugsweise C₁-C₁₀-, insbesondere C₁-C₄-Alkyl, -O-Alkyl oder -S-Alkyl, C₆-C₁₄-, vorzugsweise C₆-C₁₀-Aryl, -O-Aryl oder -S-Aryl, C₅-C₁₃-, vorzugsweise C₅-C₁₀-Heteroaryl, C₇-C₂₆-, vorzugsweise C₇-C₁₃-Alkylaryl, -O-Alkylaryl oder -S-Alkylaryl substituierten oder unsubstituierten aliphatischen Ring bilden, der ein oder mehrere Heteroatome beinhalten kann, dieser aliphatische Ring kann insbesonders durch -S-, -O-, -N(H)- unterbrochen sein.
   Von den Resten R¹ bis R⁴ können jeweils zwei benachbarte Reste zusammen mit dem Grundkörper einen weiteren 5-6-gliedrigen, bevorzugt 6-gliedrigen, mit Halogen, NO₂, CF₃, CN, C₁-C₂₀-, vorzugsweise C₁-C₁₀-, insbesondere C₁-C₄-Alkyl, -O-Alkyl oder -S-Alkyl, C₆-C₁₄-, vorzugsweise C₆-C₁₀-Aryl, -O-Aryl oder -S-Aryl, C₅-C₁₃-, vorzugsweise C₅-C₁₀-Heteroaryl, C₇-C₂₆-, vorzugsweise C₇-C₁₃-Alkylaryl, -O-Alkylaryl oder -S-Alkylaryl substituierten oder unsubstituierten aromatischen Ring bilden, der ein oder mehrere Heteroatome beinhalten kann oder mit einem weiteren aromatischen Kern annelliert sein kann.
R⁶ bedeutet -O- oder -N(R⁸)-,
   wobei R⁸ Wasserstoff oder C₁-C₁₂-, vorzugsweise C₁-C₄-Alkyl, insbesondere aber Wasserstoff bedeutet.
R⁷ bedeutet H, C₁-C₃₀-Alkyl, vorzugsweise C₁-C₁₆-Alkyl, insbesondere C₁-C₅-Alkyl, C₃-C₁₂-Cycloalkyl, vorzugsweise C₅-C₆-Cycloalkyl; mit Halogen, NO₂, CN, CF₃, O-C₁-C₂₀-, vorzugsweise O-C₁-C₁₀-, insbesondere O-C₁-C₄-Alkyl, O-C₆-C₁₄-, vorzugsweise O-C₆-C₁₀-Aryl, oder O-C₇-C₂₆-, vorzugsweise O-C₇-C₁₃-Arylalkyl substituiertes oder unsubstituiertes C₆-C₁₄-Aryl; mit Halogen, NO₂, CF₃, O-C₁-C₂₀-, vorzugsweise O-C₁-C₁₀-, insbesondere O-C₁-C₄-Alkyl, O-C₆-C₁₄-, vorzugsweise O-C₆-C₁₀-Aryl, oder O-C₇-C₂₆-, vorzugsweise O-C₇-C₁₃-Arylalkyl substituierte oder unsubstituierte C₇-C₃₀-Arylalkyl; oder einen heteroaromatischen Rest mit 5 - 15 C-Atomen bedeutet.

Bei Verwendung in organischem Material können sie dem zu stabilisierenden Material vor, während oder nach der Polymerisation in fester, geschmolzener, in Lösungsmitteln gelöster Form oder auch als Masterbatch zugegeben werden. Bei dem Zusatz als Feststoff eignen sich die Verbindungen der Formel (I) besonders in fein verteilter Form. Ein Masterbatch eignet sich besonders gut, wenn es den neuen Stabilisator in einer Konzentration von 1 bis 80 %, vorzugsweise aber von 5-30 Gew.-% enthält, der Rest im Masterbatch ist ein mit dem zu stabilisierenden Polymer verträgliches Polymer. Besonders eignet sich die Einarbeitung in gelöster Form, wobei die Lösungen den neuen Stabilisator z. B. in 5 - 80 Gew.-%iger Konzentration enthalten können. Sowohl die Lösung, als auch das Masterbatch können zusätzlich noch weitere Stabilisatoren oder Effektstoffe, z. B. weitere UV-Absorber, Lichtschutzmitteln auf Basis von sterisch gehinderten Aminen, Quenchern, Antioxidantien, Pigmente, Säurefänger oder Füllstoffe, enthalten. Die neuen Stabilisatoren werden vorzugsweise so eingesetzt, daß sie im zu stabilisierenden Polymer in einer Konzentration von 0.001 bis 5 Gew.-%, vorzugsweise von 0.02 bis 2 Gew.-%, bezogen auf das organische Material, entweder alleine oder in Kombination mit weiteren Additiven enthalten sind.

Unter organischem Material sind beispielsweise Vorprodukte für Kunststoffe, Anstrichmittel, Lacke und Öle, insbesondere jedoch Kunststoffe, Anstrichmittel, Lacke und Öle selbst zu verstehen.

Im besonderen eignen sich die Stabilisatoren der allgemeinen Formel (I) zum Stabilisieren von Folien, Fasern, Bändchen, Multifilamenten, Geweben, Extrusions-, Blasform-, Spritzguß-, Tiefziehartikeln, Pulverlacken, Druckfarben, Tonerfarben, photographischem Material, Pigmenten, Holzbeizen, Leder, Anstrichfarben für Gebäude, Schutzanstrichen für Stahlkonstruktionen, Schmierölen, Maschinenölen, Bitumen, Asphalt. Sie eignen sich auch für kosmetische oder pharmazeutische Anwendungen, in denen der UV-absorbierende Charakter der Verbindungen der Formel (I) von Vorteil ist. Verbindungen der Formel (I) eignen sich auch zur Verwendung in UV-Filtern, insbesonders wenn diese Verbindungen in polymerem Material eingebettet oder gelöst sind.

Die erfindungsgemäßen Stabilisatoren der Formel (I) können auch in vorteilhafter Weise in Kombinationen mit weiteren Stabilisatoren eingesetzt werden. Das Resultat dieser neuen Kombinationen sind Mischungen mit einem verbesserten Eigenschaftsprofil gegenüber den Einzelkomponenten, wie z. B. synergistische Effekte in der Lichtschutzwirkung.
Gegenstand der vorliegenden Erfindung ist außerdem ein gegen die Einwirkung von Licht, Sauerstoff und Wärme stabilisiertes organisches Material, insbesondere Kunststoffe, Anstrichmittel, Lacke und Öle, welches Verbindungen der Formel (I) in den oben angegebenen Konzentrationen enthält.

Beispiele für derartige Materialien sind in der deutschen Patentanmeldung Nr. 19 719 944.5 auf den Seiten 44 bis 50 beschrieben, worauf hier ausdrücklich Bezug genommen wird.

Das durch die Verbindungen der Formel (I) oder durch eine geeignete Kombination mit diesen Verbindungen stabilisierte organische Material kann gegebenenfalls noch weitere Additive enthalten, beispielsweise Antioxidantien, Lichtstabilisierungsmittel, Metalldesaktivatoren, antistatische Mittel, flammhemmende Mittel, Gleitmittel, Nukleierungsmittel, Säurefänger (basische Costabilisatoren), Hydrotalcite, Pigmente und Füllstoffe. Geeignete Additive, die neben den Verbindungen (I) zugesetzt werden, sind beispielsweise Verbindungen auf der Basis sterisch gehinderter Amine oder sterisch gehinderter Phenole oder Schwefel oder Phosphor enthaltende Costabilisatoren.

Die Additive der allgemeinen Formel (I) oder Kombinationen von (I) mit weiteren Additiven werden nach den allgemein üblichen Methoden in das organische Material, vorzugsweise in das Polymere eingearbeitet. Die Einarbeitung kann beispielsweise durch Einmischen oder Aufbringen der Verbindungen und gegebenenfalls weiterer Additive in oder auf das Polymere unmittelbar vor, während oder nach der Polymerisation oder in die Polymerschmelze vor oder während der Formgebung erfolgen. Auch durch Aufbringen der gelösten oder dispergierten Verbindungen auf das Polymere direkt oder Einmischen in eine Lösung, Suspension oder Emulsion des Polymeren, gegebenenfalls unter nachträglicher Verdunstung des Lösemittels, kann die Einarbeitung erfolgen. Bei dem Zusatz als Feststoff eignen sich die Verbindungen der Formel (I) besonders in fein verteilter Form. Die Verbindungen sind auch wirksam, wenn sie in ein bereits granuliertes Polymer nachträglich in einem gesonderten Verarbeitungsschritt eingebracht werden.

### Experimenteller Teil

### Allgemeine Vorbemerkungen zur Herstellung der neuen Lichtschutzmittel

Die erfindungsgemäß eingesetzten Verbindungen 1 bis 9 wurden nach dem Stand der Technik hergestellt, insbesonders nach der u. a. in Organic Syntheses Coll. Vol. III, S. 274 und in Houben-Weyl, Heterarene II, Teil 1, Band E7a, S. 343 ff beschriebenen Methoden, d. h. durch die thermische Cyclisierung von geeignet substituierten (Arylamino-methylen)-malonsäure-dialkylestern in einem hochsiedenden Lösungsmittel. Die cyclisierbaren (Arylamino-methylen)-malonsäuredialkylester wurden durch Kondensation von primären Aminen mit Ethoxymethylenmalonsäure-dialkylestern hergestellt, wie in Houben-Weyl, Heterarene II, Teil 1, Band E7a, S. 345 beschrieben. Die cyclisierbaren S-Alkyl-substituierten (Arylaminomethylen)-malonsäure-dialkylester für die Synthese von 4-Hydroxychinolin-3-carbonsäureester mit dem -S-Alkyl-Substituent, insbesonders dem -S-CH₃-Substituent, in 2-Position können analog der in Houben-Weyl, Heterarene II, Teil 1, Band E7a, S. 376 publizierten Methode, insbesonders durch Reaktion von Arylthioisocyanat mit dem Natriumsalz der Malonsäure und anschließender Umsetzung mit Alkyliodid hergestellt werden. Die Vorstufen für die Derivate mit dem -CN-Substituent in 2-Position wurden nach der von S. E. J. Glue, I. T. Kay in Synthesis (1977), 607-8 beschriebenen Methode hergestellt.

### Beispiele 1 - 7:

0.2 mol Ethoxymethylen-malonsäure-diethylester (Hüls AG, Marl) werden mit 0.2 mol eines am aromatischen Kern geeignet substituierten Anilin-Derivates in 200 ml Xylol etwa 20 h lang unter DC-Kontrolle (DC = Dünnschichtchromatographie) unter Rückfluß gerührt. Das dabei abgespaltene Ethanol wird dabei zusammen mit einer kleinen Menge an Xylol kontinuierlich abdestilliert. Die Innentemperatur steigt zuerst auf etwa 65 °C, nach 10 h Rückfluß auf etwa 100 °C, später dann auf bis zu 112 °C. Nach Ende der Umsetzung (DC-Kontrolle) werden die flüchtigen Bestandteile bei 90 °C und 10 mbar entfernt. Der (Arylamino-methylen)-malonsäure-diethylester fällt dabei in ausreichender Reinheit an (¹H-NMR), die Ausbeuten liegen bei 90 - 95%. 0.2 mol des so erhaltenen Arylaminoethylens werden in 190 ml siedenden Diphenylether (247 °C) eingetragen; das sofort entstehende Ethanol wird dabei kontinuierlich abdestilliert. Nach 50 min. wird die entstandene Suspension auf 40 °C abgekühlt und mit 250 ml Hexan versetzt. Der Niederschlag wird 12 h bei 20 °C nachgerührt, abgesaugt, mit 50 ml Hexan nachgewaschen und im Vakuumtrockenschrank bei 50 °C von anhaftendem Hexan befreit. Ausbeute und Schmelzpunkt der jeweiligen Produkte sind in Tabelle 1 zusammengefaßt.

### Beispiel 8:

80.0 g (0.5 mol) Malonsäurediethylester werden in 400 ml DMF gelöst und unter Stickstoff bei etwa 20 °C mit 15.2 g (0.505 mol) NaH (80%ig in Paraffin) unter Rühren portionsweise versetzt. Die Mischung wird zur Vervollständigung der Umsetzung 1 h bei 50 °C gerührt. Nach Abkühlen auf Raumtemperatur werden 78.1 g (0.55 mol) Methyliodid (in 50 ml DMF gelöst) so zugetropft, daß die Innentemperatur nicht über 40 °C steigt, wobei ein hellgelber Niederschlag ausfällt. Die Suspension wird 20 h bei 20 °C gerührt und anschließend auf ein Gemisch von 300 ml Wasser und 300 ml Methylenchlorid gegossen. Die organische Phase wird abgetrennt, mit 20 g Natriumsulfat getrocknet, von diesem abgetrennt und mit 5 g gekörnter Aktivkohle bei 20 °C intensiv gemischt. Nach Filtration werden die flüchtigen Bestandteile bei 90 °C und 10 mbar entfernt. Ausbeute: 147.5 g (96%), gelbes Öl.
Das so erhaltene gelbe Öl wird unter Stickstoff in siedendes o-Dichlorbenzol eingetragen (etwa 166 °C). Das sofort entstehende Ethanol wird dabei kontinuierlich abdestilliert. Nach Beendigung der Zugabe wird die Lösung 2 h bei 166 °C gerührt und anschließend auf 20 °C abgekühlt. Bei 120 °C und 10 mbar werden die flüchtigen Bestandteile entfernt, es verbleibt ein gelbkristallines Rohprodukt (121.5 g), das mit Heptan umkristallisiert wird. Nach Umkristallisation werden 91.1 g (72.2 %) eines gelbkristallinen Feststoffes isoliert (Fp.: 82-83 °C).

### Beispiel 9:

27.6 g (0.2 mol) Phenylisothiocyanat (c=98%) werden unter Stickstoff in 400 ml abs. Methanol vorgelegt. Bei 20 °C werden unter Rühren 13.4 g (0.2 mol) KCN zugegeben, es entsteht eine leicht gelbe Suspension. Die Reaktionsmischung wird auf 45 °C aufgeheizt, wobei die festen Bestandteile in Lösung gehen. Bei 45 °C werden 35.4 g (0.2 mol) Chlormalonsäuredimethylester (c=94%ig) rasch zugegeben. Die orange Lösung wird 3 h bei 20 °C gerührt, es bildet sich ein heller Niederschlag. Die Suspension wird filtriert; der Niederschlag wird verworfen und die Lösung bei 90 °C und 10 mbar von den flüchtigen Bestandteilen befreit. Das verbleibende Rohprodukt wird mit 200 ml Methyl-tert.-butylether aufgenommen und zweimal mit je 200 ml Wasser ausgeschüttelt. Die organische Phase wird mit Natriumsulfat getrocknet und erneut bei vermindertem Druck von den flüchtigen Bestandteilen befreit. Das Produkt wird als oranges Öl isoliert (51.9 g, 99.8%).

Das so erhaltene orange Öl wird in o-Dichlorbenzol gelöst und unter Rühren auf Rückfluß (168 °C) gebracht. Das dabei entstehende Methanol wird kontinuierlich abdestilliert. Nach 4 h Rühren bei 163 °C (DC-Kontrolle) wird die Lösung abgekühlt. Bei 120 °C und 10 mbar werden die flüchtigen Bestandteile entfernt. Der braune Rückstand wird mit 50 ml Diisopropylether ausgerührt, die Suspension wird erneut im Vakuum von den flüchtigen Bestandteilen befreit. Das Rohprodukt wird aus 50 ml Ethanol umkristallisiert. Ausbeute: 10.4 g (22.8%), ockergelbe Kristalle, Fp. 181-183 °C.

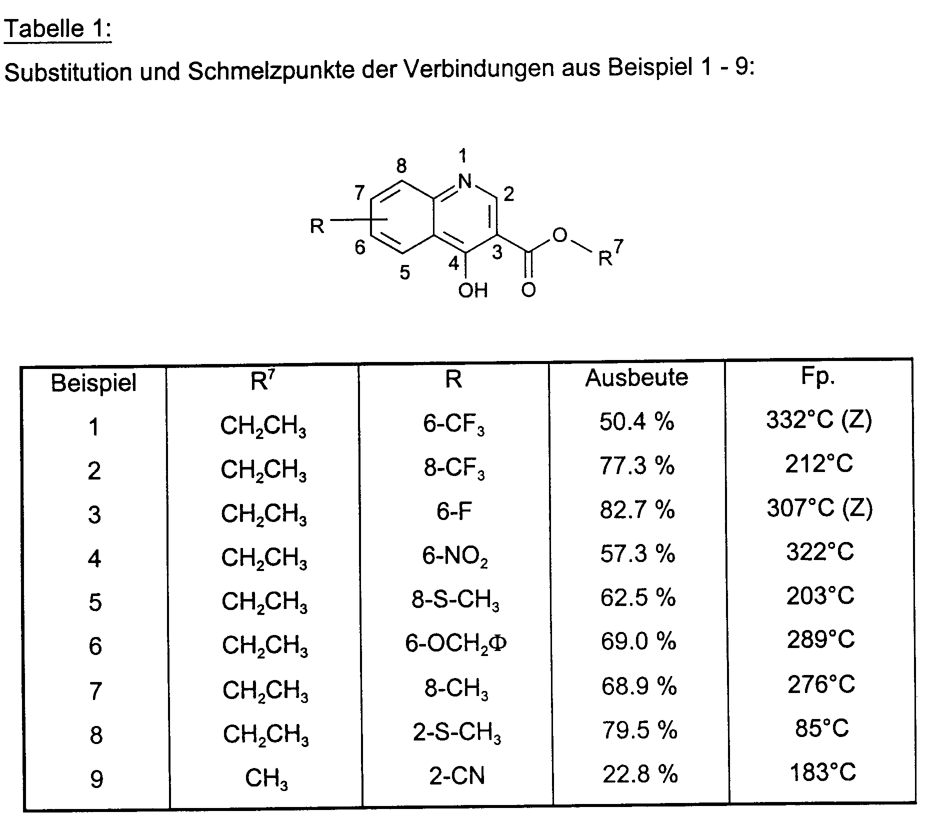

**Tabelle 2**

| Charakterisierung von vier ausgewählten Beispielen im UV-Spektrum | | |
|---|---|---|
| Verbindung aus Beispiel | Absorptionsmaximum | Extinktionskoeffizient |
| 2 | 300 nm (s) | 9302 |
| | 321 nm (vs) | 10056 |
| | 332 nm (vs) | 10316 |
| 5 | 325 nm (vs) | 14130 |
| | 336 nm (s, sh) | -- |
| 7 | 313 nm (vs) | 15978 |
| | 324 nm (s, sh) | -- |
| 8 | 268 nm (vs) | 20259 |
| | 325 nm (s) | 8734 |

**Tabelle 3**

| Flüchtigkeit von drei ausgewählten Beispielen | | | |
|---|---|---|---|
| Verbindung | 1 | 4 | 6 |
| Flüchtigkeit* | 49 % | 4.3 % | 24.5 % |

| | | | |
|---|---|---|---|
| *Messung der Flüchtigkeit unter Stickstoff; Einwaage: 500 mg; Pfännchen: Pt; Oberfläche: 3 cm²; Aufheizung: 120 °C/h (Aufheizen auf 300 °C, dann 10 min. bei 300 °C weiterheizen). | | | |

### Beispiele 10 - 14: Lichtstabilisierende Wirkung in Polypropylenfolien

100 Gewichtsteile unstabilisiertes Polypropylen (®Hostalen PPK, Hoechst AG) wurden zusammen mit 0.1 Gewichtsteilen Calciumstearat (Fa. Greven), 0.05 Gewichtsteilen Pentaerythrityl-tetrakis-[3-(3,5-di-tert.butyl-4-hydroxyphenyl)-propionat (®Hostanox O 10, Hoechst AG) und 0.1 Gewichtsteilen des zu prüfenden Stabilisators gemischt und einmal granuliert. Das Granulat wird zu Spritzgußplatten der Dicke 1.0 mm verarbeitet, die in einem Schnellbewitterungsgerät (®Xenotest 1200) belichtet wurden. Als Kriterium der Stabilität der Spritzplatte wurde die Veränderung der Oberflächenversprödung herangezogen. Der Grad der Oberflächenversprödung wurde dabei mit Werten zwischen 0 und 6 beurteilt; dabei entspricht ein Wert von 0 keiner sichtbaren Veränderung und der Wert von 6 einer sehr starken Oberflächenversprödung. Zu Vergleichszwecken wurde eine Spritzplatte unter den gleichen Bedingungen, jedoch ohne den Zusatz eines erfindungsgemäßen Stabilisators getestet. Die Versuchsergebnisse sind in Tabelle 4 zusammengestellt:

**Tabelle 4**

| Veränderung der Oberflächenversprödung erfindungsgemäß stabilisierter PP-Spritzplatten | | |
|---|---|---|
| Beispiel Nr. | Stabilisator | Oberflächenversprödung nach 460 h |
| 10 | kein Stabilisator | 2 |
| 11 | Stabilisator **2** | 0 |
| 12 | Stabilisator **5** | 1 |
| 13 | Stabilisator **7** | 0 |
| 14 | Stabilisator **8** | 0 |

Tabelle 4 unterstreicht die Lichtschutzwirkung der erfindungsgemäßen Stabilisatoren in Polypropylen.

## Patentansprüche

1. Verwendung von Verbindungen der Formel (I) 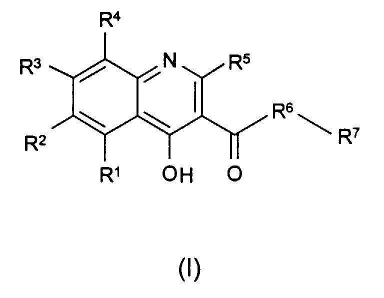 wobei
R¹ bis R⁵ unabhängig voneinander Halogen, H, NO₂, CF₃, CN;
C₁-C₂₀-Alkyl, -S-Alkyl oder -O-Alkyl; C₆-C₁₄-Aryl, -S-Aryl oder -O-Aryl; C₅-C₁₃-Heteroaryl; C₇-C₂₆-Alkylaryl, -S-Alkylaryl oder -O-Alkylaryl bedeuten, und wobei
zwei der Reste R¹ bis R⁴ zusammen mit dem Grundkörper einen 5-12-gliedrigen, mit Halogen, NO₂, CN, CF₃; C₁-C₂₀-Alkyl, -O-Alkyl oder -S-Alkyl; C₆-C₁₄-Aryl, -S-Aryl oder -O-Aryl; C₅-C₁₃-Heteroaryl; C₇-C₂₆-Alkylaryl, -S-Alkylaryl oder -O-Alkylaryl substituierten oder unsubstituierten aliphatischen Ring bilden können, der ein oder mehrere Heteroatome beinhalten kann,
und wobei von den Resten R¹ bis R⁴ jeweils zwei benachbarte Reste zusammen mit dem Grundkörper einen 5-6-gliedrigen, mit Halogen, NO₂, CF₃, CN; C₁-C₂₀-Alkyl, -O-Alkyl oder -S-Alkyl; C₆-C₁₄-Aryl, -S-Aryl oder -O-Aryl; C₅-C₁₃-Heteroaryl; C₇-C₂₆-Alkylaryl, -S-Alkylaryl oder -O-Alkylaryl substituierten oder unsubstituierten aromatischen Ring bilden können, der ein oder mehrere Heteroatome beinhalten kann oder mit einem weiteren aromatischen Kern annelliert sein kann,
R⁶ -O- oder -N(R⁸)-, wobei R⁸ Wasserstoff oder C₁-C₁₂-Alkyl bedeutet,
R⁷ H, C₁-C₃₀-Alkyl, C₃-C₁₂-Cycloalkyl; mit Halogen, NO₂, CF₃, O-C₁-C₂₀-Alkyl, O-C₆-C₁₄-Aryl oder O-C₇-C₂₆-Arylalkyl substituiertes oder unsubstituiertes C₆-C₁₄-Aryl; mit Halogen, NO₂, CF₃, O-C₁-C₂₀-Alkyl, O-C₆-C₁₄-Aryl oder O-C₇-C₂₆Arylalkyl substituiertes oder unsubstituiertes C₇-C₃₀-Arylalkyl, oder einen heteroaromatischen Rest mit 5 - 15 C-Atomen, bedeutet,
als Stabilisator für organisches Material gegen den schädlichen Einfluß von Licht, Sauerstoff und Wärme.

2. Verwendung gemäß Anspruch 1, dadurch gekennzeichnet, daß
R¹ bis R⁵ unabhängig voneinander Halogen, H, NO₂, CF₃, CN;
C₁-C₄-Alkyl, -S-Alkyl oder -O-Alkyl; C₆-C₁₀-Aryl, -S-Aryl oder -O-Aryl; C₅-C₁₃-Heteroaryl; C₇-C₁₃-Alkylaryl, -S-Alkylaryl oder -O-Alkylaryl bedeuten, und wobei
zwei der Reste R¹ bis R⁴ zusammen mit dem Grundkörper einen 5-6-gliedrigen, mit Halogen, NO₂, CN, CF₃; C₁-C₄-Alkyl, -O-Alkyl oder -S-Alkyl; C₆-C₁₀-Aryl, -S-Aryl oder -O-Aryl; C₅-C₁₃-Heteroaryl; C₇-C₁₃-Alkylaryl, -S-Alkylaryl oder -O-Alkylaryl substituierten oder unsubstituierten aliphatischen Ring bilden können, der ein oder mehrere Heteroatome beinhalten kann,
und wobei von den Resten R¹ bis R⁴ jeweils zwei benachbarte Reste zusammen mit dem Grundkörper einen 6-gliedrigen, mit Halogen, NO₂, CF₃, CN; C₁-C₄-Alkyl, -O-Alkyl oder -S-Alkyl; C₆-C₁₀-Aryl, -S-Aryl oder -O-Aryl; C₅-C₁₃-Heteroaryl; C₇-C₁₃-Alkylaryl, -S-Alkylaryl oder -O-Alkylaryl substituierten oder unsubstituierten aromatischen Ring bilden können, der ein oder mehrere Heteroatome beinhalten kann oder mit einem weiteren aromatischen Kern annelliert sein kann,
R⁶ -O- oder -N(R⁸)-, wobei R⁸ Wasserstoff oder C₁-C₄-Alkyl bedeutet,
R⁷ H, C₁-C₅-Alkyl, C₅-C₈-Cycloalkyl; mit Halogen, NO₂, CF₃, O-C₁-C₄-Alkyl, O-C₆-C₁₀-Aryl oder O-C₇-C₁₃-Arylalkyl substituiertes oder unsubstituiertes C₆-C₁₀-Aryl; mit Halogen, NO₂, CF₃, O-C₁-C₄-Alkyl, O-C₆-C₁₀-Aryl oder O-C₇-C₁₃-Arylalkyl substituiertes oder unsubstituiertes C₇-C₃₀-Arylalkyl, oder einen heteroaromatischen Rest mit 5 - 15 C-Atomen bedeutet.

3. Verwendung gemäß Anspruch 1, dadurch gekennzeichnet, daß
R¹ bis R⁵ unabhängig voneinander Halogen, H, NO₂, CF₃, CN; C₁-C₄-Alkyl, -S-Alkyl oder -O-Alkyl; C₆-C₁₀-Aryl, -S-Aryl oder -O-Aryl; C₇-C₁₃-Alkylaryl, -S-Alkylaryl oder -O-Alkylaryl bedeuten,
R⁶ -O- oder -N(R⁸)-, wobei R⁸ Wasserstoff bedeutet,
R⁷ H, C₁-C₅-Alkyl, C₈-C₁₀-Aryl, C₇-C₃₀-Arylalkyl, oder einen heteroaromatischen Rest mit 5 - 15 C-Atomen bedeutet.

4. Verwendung gemäß Anspruch 1, dadurch gekennzeichnet, daß es sich bei dem organischen Material um Kunststoffe, Anstrichmittel, Öle, Lacke oder Vorprodukte davon handelt.

5. Verwendung gemäß Anspruch 1, dadurch gekennzeichnet, daß es sich bei dem organischen Material um pharmazeutische oder kosmetische Produkte handelt.

6. Verfahren zum Stabilisieren von organischem Material gegen den durch Licht, Strahlung, Wärme und Sauerstoff verursachten Abbau, dadurch gekennzeichnet, daß man dem zu stabilisierenden Material eine Verbindung der Formel (I) nach Anspruch 1 in einer Konzentration von 0.001 - 5 Gew.%, bevorzugt von 0.1 - 2.0 Gew.% zusetzt.

7. Stabilisiertes organisches Material, welches mindestens eine der Verbindungen gemäß Anspruch 1 enthält.

8. Stabilisiertes organisches Material gemäß Anspruch 7 welches als weitere Additive Antioxidantien, Lichtstabilisatoren, Metalldesaktivatoren, Antistatika, flammhemmende Mittel, Pigmente, Säurefänger oder Füllstoffe, enthält.

9. Stabilisiertes organisches Material gemäß Anspruch 7 oder 8, dadurch gekennzeichnet, daß es sich um ein Polymeres handelt.
